# EUROPÄISCHE PATENTSCHRIFT

(11) **EP 2 974 746 B1**
(45) Veröffentlichungstag und Bekanntmachung des Hinweises auf die Patenterteilung: **10.11.2021**
(21) Anmeldenummer: 15162197.6
(22) Anmeldetag: 01.04.2015
(51) Int. Cl.: A61L 2/025, A61L 2/22, B08B 3/12, H02J 5/00, A47L 15/13

(54) **REINIGUNGSAUTOMAT MIT ULTRASCHALLMODUL FÜR MEDIZINISCHE INSTRUMENTE**
CLEANING MACHINE WITH ULTRASOUND MODULE FOR MEDICAL INSTRUMENTS
AUTOMATE DE NETTOYAGE AVEC MODULE À ULTRASONS POUR INSTRUMENTS MÉDICAUX

(30) Priorität: 02.04.2014 DE 102014104618
(43) Veröffentlichungstag der Anmeldung: 20.01.2016
(73) Patentinhaber: Miele & Cie. KG, 33325 Gütersloh (DE)
(72) Erfinder: Malec, Tobias, 33739 Bielefeld (DE); Tosberg, Christian, 33613 Bielefeld (DE)

(56) Entgegenhaltungen:
- EP-A1- 0 286 524
- EP-A1- 1 774 979
- EP-A1- 2 932 984
- EP-A2- 0 396 363
- EP-A2- 2 769 696
- WO-A2-2007/011520
- DE-A1- 2 019 346
- DE-U1-202004 007 952
- DE-U1-202006 020 523
- FR-A1- 2 846 865
- US-A- 5 203 362
- US-A- 5 377 709
- None

## Beschreibung

Die Erfindung betrifft einen Reinigungsautomaten für medizinische Instrumente, bei dem in einem Spülraum mindestens ein Sprüharm zum Versprühen einer Reinigungsflüssigkeit angeordnet ist. Die Erfindung betriff weiterhin ein Ultraschallmodul für einen Reinigungsautomaten und ein System aus einem Reinigungsautomaten und einem Ultraschallmodul.

In Krankenhäusern oder Arztpraxen werden medizinische Instrumente, die zu Operationszwecken verwendet wurden, nach der Benutzung aufbereitet. Die Aufbereitung umfasst hierbei eine Reinigung, Desinfektion, Trocknung und ggf. eine sich anschließende Sterilisation. Die Schritte des Reinigen, Desinfizieren und Trocknen werden üblicherweise in einem Reinigungs- und Desinfektionsautomaten, abgekürzt auch als Reinigungsautomat bezeichnet, durchgeführt. Dabei haben sich mit Geschirrspülmaschinen vergleichbar arbeitende Reinigungsautomaten der eingangs genannten Art mit mindestens einem Sprüharm, über den eine Reinigungsflüssigkeit im Spülraum versprüht wird, etabliert.

Aufgebaut sind die Reinigungsautomaten häufig in Form von Schleusen, so dass Instrumente aus einem nicht desinfizierten Bereich durch den Reinigungsautomaten als Schleuse in einen desinfizierten Bereich transferiert werden.

Aufgrund von teilweise sehr hartnäckigen Verschmutzungen können manche der medizinischen Instrumente in einem derartigen Reinigungsautomaten nicht ausreichend gereinigt werden. Bei diesen Instrumenten erfolgt meist eine manuelle Vorreinigung, um die gröbsten und hartnäckigsten Verunreinigungen vorab zu lösen.

Die manuelle Vorreinigung erfolgt in der Regel in einem separaten Ultraschallbad, gefolgt von einem anschließenden Abbürsten. Diese Bearbeitungsschritte sind sehr zeitintensiv und binden entsprechend Personal.

Aus der Druckschrift DE 44 44 148 A1 ist eine Haushaltsgeschirrspülmaschine bekannt, bei der eine Ultraschallreinigung in die Geschirrspülmaschine integriert ist. Zur Ultraschallreinigung wird der Spülraum der Geschirrspülmaschine, in dem sich das zu reinigende Geschirr befindet, mit einer Reinigungsflüssigkeit geflutet, über die Ultraschallwellen auf das zu reinigende Geschirr übertragen werden. Zur Erzeugung der Ultraschallwellen sind Ultraschallerreger außen an einer Seitenwand des Spülraums angeordnet. Hierbei ist nachteilig, dass aufgrund der Flutung des der gesamten Spülraums eine große Wassermenge benötigt wird. Um diese Menge nicht bei jedem Reinigungsvorgang als Frischwasser bereitstellen zu müssen, ist ein entsprechend großer Zwischenbehälter vorgesehen, in dem die zur Flutung des Spülraums benötigte Reinigungsflüssigkeit für eine Wiederverwendung zwischengelagert werden kann. Dieses schränkt die praktische Verwendbarkeit des Systems auf relativ kleine Geschirrspülmaschinen ein. Das System ist für einen Reinigungsautomat der eingangs genannten Art mit einem üblicherweise sehr großen Spülraumvolumen ungeeignet.

Bei Reinigungsautomaten der eingangs genannten Art ist zur Integration einer Ultraschallreinigung in den Reinigungsautomaten bekannt, eine größere Menge Reinigungsflüssigkeit in einem unteren wannenförmigen Bereich des Reinigungsautomaten vorzuhalten, der mit Ultraschallwellen beaufschlagt werden kann. In den Reinigungsautomaten wird ein Spülgutwagen mit mehreren Ebenen eingeschoben, in dem das Spülgut zur Reinigung gelagert wird. Der eingeschobene Spülgutwagen kann abgesenkt werden, wodurch die unteren zwei Ebenen des Spülgutwagens in das Ultraschallbad eintauchen und dort vorgereinigt werden können. Nachteilig ist auch hier, dass eine große Menge an Reinigungsflüssigkeit im Reinigungsautomaten vorgehalten werden muss, um genügend Volumen zum Eintauchen zumindest einer oder mehrerer der unteren Ebenen des Spülgutwagens zu ermöglichen. Der wannenförmige untere Bereich nimmt entsprechend einen großen Bauraum ein, was entweder die Baugröße insgesamt vergrößert oder zu Lasten der Aufnahmekapazität des Reinigungsautomaten geht.

Die DE 2019346 A1 offenbart einen Spülautomaten mit einem Spülraum, in dem ein Sprüharm zum Versprühen einer Reinigungsflüssigkeit angeordnet ist, und mit einem Behälter mit einem darin eingebautem Schwingungserzeuger zur Beaufschlagung der Spülflüssigkeit im Behälter mit Ultraschall.

Die EP 1774979 A1 und die FR 2846865 A1 offenbaren einen Reinigungsautomat mit einem Sprüharm zum Versprühen einer Reinigungsflüssigkeit, wobei innerhalb des Sprüharms ein Ultraschallemitter angeordnet ist, mit dem die den Sprüharm durchströmende Reinigungsflüssigkeit mit Ultraschall beaufschlagt werden kann.

Es ist eine Aufgabe der vorliegenden Erfindung, einen Reinigungsautomaten der eingangs genannten Art mit einer integrierten Ultraschallreinigungsmöglichkeit zu schaffen, ohne dass sich der Wasserverbrauch signifikant erhöht und ohne dass sich die Beladungskapazität des Reinigungsautomaten verringert bzw. sich seine Baugröße vergrößert. Es ist eine weitere Aufgabe, ein Ultraschallmodul für einen Reinigungsautomaten und ein System aus einem Reinigungsautomaten und einem Ultraschallmodul bereitzustellen.

Diese Aufgabe wird durch ein System mit den Merkmalen des unabhängigen Patentanspruchs gelöst. Vorteilhafte Ausgestaltungen und Weiterbildungen sind in den abhängigen Ansprüchen angegeben.

Ein erfindungsgemäßer Reinigungsautomat zeichnet sich dadurch aus, dass er zur Aufnahme eines aus dem Spülraum entnehmbaren Ultraschallmoduls eingerichtet ist. Dadurch, dass das Ultraschallmodul nicht fest integriert in den Reinigungsautomaten ist, sondern entnehmbar gestaltet ist, kann es bedarfsweise hinzugeführt werden und verändert weder die grundsätzliche Baugröße des Reinigungsautomaten, noch seine Beladungskapazität.

In einer vorteilhaften Ausgestaltung weist der Reinigungsautomat eine Ultraschallgeneratoreinheit mit einem Ultraschallsignalgenerator zum Bereitstellen eines elektrischen Wechselspannungssignals auf, wobei der Ultraschallgenerator außerhalb des Spülraums angeordnet ist. Dadurch werden elektronische Komponenten des Ultraschallsignalgenerators vor den hohen im Spülraum herrschenden Temperaturen geschützt. Bevorzugt ist bei dem Reinigungsautomaten ein Kupplungsgegenstück zur Energieübertragung auf ein korrespondierendes Kupplungselement des entnehmbaren Ultraschallmoduls vorgesehen. Besonders bevorzugt wirkt und/oder ragt das Kupplungsgegenstück in den Spülraum hinein. Der Reinigungsautomat kann so die für den Betrieb des Ultraschallmoduls benötigte Energie an passender Stelle innerhalb des Spülraums bereitstellen.

Ein erfindungsgemäßes Ultraschallmodul zur Verwendung in einem Reinigungsautomaten für medizinische Instrumente weist eine Ultraschallwanne auf, die über eine Trennwand mit mindestens einem Ultraschallwandler gekoppelt ist, wobei der mindestens eine Ultraschallwandler in einer wasserdichten Umhausung angeordnet ist. Ein derartiges Ultraschallmodul kann bei Bedarf in den Spülraum des Reinigungsautomaten eingesetzt werden.

In einer vorteilhaften Ausgestaltung des Ultraschallmoduls ist die Ultraschallwanne nach oben offen. Sie füllt sich dadurch automatisch durch die von den Sprüharmen des Reinigungsautomaten versprühte Reinigungsflüssigkeit. In einer alternativen Ausgestaltung kann die Ultraschallwanne auch nach oben geschlossen sein, wobei dann an der Ultraschallwanne ein Wasserzulauf angeordnet ist. Hierbei kann ein Füllen der Ultraschallwanne mit Reinigungsflüssigkeit beispielsweise gezielt über einen Wasserauslass erfolgen, der im Spülraum des Reinigungsautomaten vorgesehen ist und der mit dem Wasserzulauf der Ultraschallwanne gekoppelt wird.

In einer weiteren vorteilhaften Ausgestaltung des Ultraschallmoduls ist in einem unteren Bereich der Ultraschallwanne ein Ablauf angeordnet, wodurch ein möglichst vollständiges Entleeren der Ultraschallwanne erreicht wird. Dabei kann der Wannenboden der Ultraschallwanne beispielsweise schräg ausgebildet, so dass es einen tiefsten Punkt der Ultraschallwanne gibt, in dem vorteilhafterweise der Ablauf angeordnet ist. Der Ablauf kann in einem Ausführungsbeispiel durch eine permanent geöffnete Bohrung gebildet sein. In diesem Fall reguliert sich die Füllhöhe der Reinigungsflüssigkeit ohne weiteres durch ein Gleichgewicht zwischen der Menge an Reinigungsflüssigkeit, die von oben in die Ultraschallwanne hineintropft, und der ablaufenden Menge. Gleichzeitig findet ein ständiger Austausch der Reinigungsflüssigkeit statt, wodurch eine optimale Reinigungswirkung erzielt wird. Bei einer alternativen Ausgestaltung ist ein elektromagnetisch oder thermisch betätigtes Ventil vorgesehen, um den Ablauf gezielt öffnen oder schließen zu können.

In einer vorteilhaften Ausgestaltung weist das Ultraschallmodul ein Kupplungselement zur Energieübertragung von einem korrespondierenden Kupplungsgegenstück des Reinigungsautomaten auf. Auf diese Weise kann das Ultraschallmodul mit dem Ultraschallsignalgenerator verbunden werden bzw. zum Entnehmen von diesem getrennt werden.

Ein erfindungsgemäßes System setzt sich aus einem derartigen Reinigungsautomaten und einem derartigen entnehmbaren Ultraschallmodul zusammen. Es ergeben sich die zuvor genannten Vorteile.

In einer vorteilhaften Ausgestaltung des Systems ist das Ultraschallmodul zur Positionierung in einem Spülgutwagen eingerichtet, der für den Reinigungsvorgang in den Reinigungsautomaten eingeschoben wird. So kann, der üblichen Praxis folgend, normal verschmutztes Spülgut in den Spülgutwagen eingeräumt werden und zusätzlich gleichzeitig die durch die Ultraschallbehandlung intensiver zu reinigende Instrumente.

Die Erfindung wird nachfolgend anhand eines Ausführungsbeispiels mithilfe von zwei Figuren näher erläutert. Die Figuren zeigen:
- Figur 1: eine perspektivische teilgeschnittene Ansicht eines Ausführungsbeispiels eines Reinigungsautomaten mit einem eingesetzten Ultraschallmodul und
- Figur 2: eine ebenfalls perspektivische und teilgeschnittene Ansicht des Ultraschallmoduls aus Figur 1.

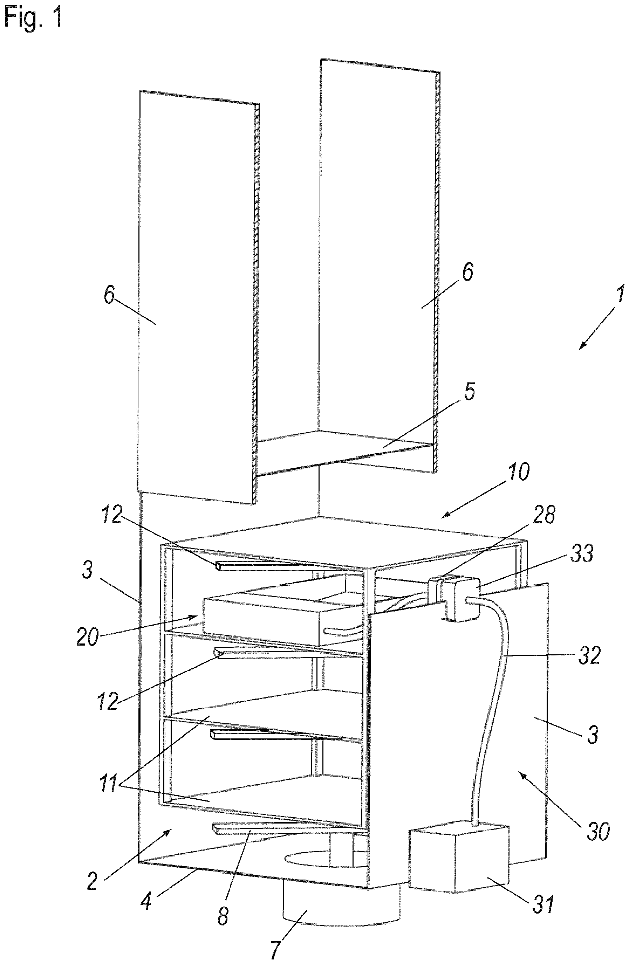

Figur 1 zeigt in einer perspektivischen teilgeschnittenen Darstellung einen Reinigungsautomat 1 zur Reinigung medizinischer Instrumente. Der Reinigungsautomat 1 weist einen Spülraum 2 auf, der durch Seitenwände 3, einen Boden 4 und eine Decke 5 begrenzt ist. Nach vorne und hinten sind Türen 6 vorgesehen, in diesem Ausführungsbeispiel ausgestaltet als vertikal verfahrbare Türen. Die Anordnung von zwei sich gegenüberliegenden Türen 6 ermöglicht es, den Reinigungsautomaten 1 in Art einer Schleuse zwischen einem nicht desinfizierten Bereich und einem desinfizierten Bereich einer Arztpraxis bzw. eines Operationsbereichs einzubauen.

Im Boden 4 des Reinigungsautomaten 1 ist ein Spültopf 7 angeordnet, in dem sich im Reinigungsautomaten 1 zirkulierende Reinigungsflüssigkeit, auch Spülflotte genannt, sammelt. Die Reinigungsflüssigkeit wird aus dem Spültopf 7 nach Filterung und ggf. weiterer Reinigung durch eine Pumpe über einen Sprüharm 8 in den Spülraum 2 zur Reinigung von eingebrachtem Spülgut gesprüht. Beispielhaft ist in der Figur 1 nur ein fest im Spülraum 2 angeordneter Sprüharm 8 dargestellt. Ein weiterer derartiger Sprüharm kann sich beispielsweise unterhalb der Decke 5 befinden.

Zur Aufnahme des Spülguts im Reinigungsautomaten 1 ist ein Spülgutwagen 10 vorgesehen, in den Spülgut auf mehreren Ebenen 11 eingeräumt werden kann. Innerhalb des Spülgutwagens 10 sind in den einzelnen Ebenen 11 weitere Sprüharme 12 angeordnet, in die ebenfalls Reinigungsflüssigkeit aus der Spülflotte des Reinigungsautomaten 1 gepumpt wird. Der übersichtlicheren Darstellung halber sind die Ebenen 11 geschlossen dargestellt. In einer Umsetzung des Reinigungsautomaten 1 bestehen die Ebenen 11 beispielsweise aus einem Drahtgittermaterial, so dass Reinigungsflüssigkeit von allen Seiten an das Spülgut gelangen kann. Zur Verbindung der weiteren Sprüharme 12 mit dem Pumpensystem des Reinigungsautomaten ist eine hier nicht dargestellte hydraulische Kupplung vorgesehen.

Der erfindungsgemäße Reinigungsautomat 1 ist zur Aufnahme eines Ultraschallmoduls 20 vorbereitet, das in einer oder mehreren der Ebenen 11 des Spülgutwagens 10 eingesetzt werden kann.

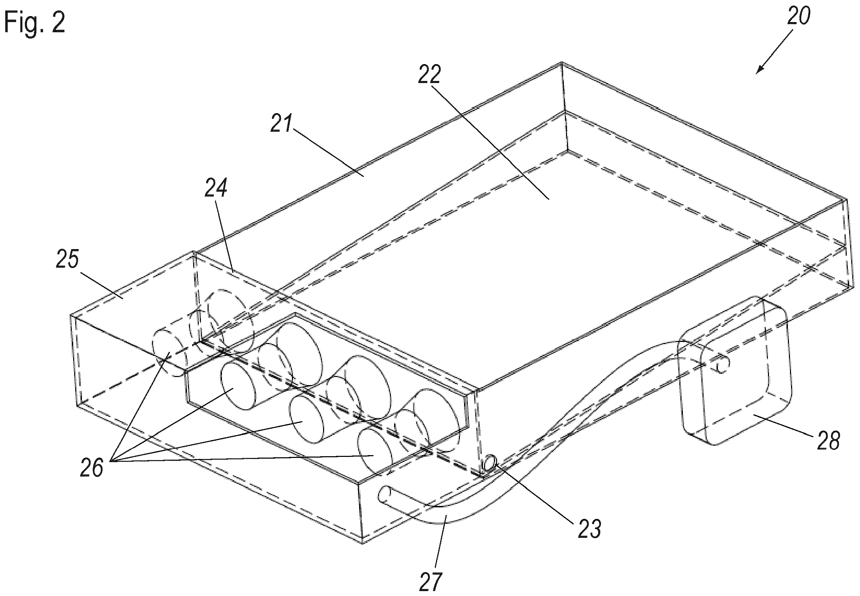

Das Ultraschallmodul 20 ist in der Figur 2 in einer ebenfalls perspektivischen und teilgeschnittenen Ansicht detaillierter dargestellt. Das Ultraschallmodul 20 umfasst eine Ultraschallwanne 21, die nach oben offen ist und nach unten eine Ablagefläche 22 für das Spülgut aufweist. Die Ablagefläche 22 kann strukturiert sein, um aufgelegten und zu reinigenden Instrumenten einen Seitenhalt zu geben. Es kann auch vorgesehen sein, dass die zu reinigenden Instrumente in hier nicht dargestellten Halterungen eingesetzt werden, um die Ablagefläche 22 selbst bzw. die Ränder der Ultraschallwanne 21 nicht zu berühren. Bei empfindlicherem Spülgut dient dieses einem Schutz vor mechanischer Beschädigung.

Die Ultraschallwanne 21 weist einen Ablauf 23 auf, der im dargestellten Beispiel aus einer oder mehreren seitlichen Bohrungen besteht. Im Betrieb des Reinigungsautomaten 1 wird Reinigungsflüssigkeit über den oder die Sprüharme 8 bzw. die weiteren Sprüharme 12 im Spülraum 2 verteilt und gelangt von oben in die Ultraschallwanne 21. Der Durchmesser des Ablaufs 23 ist so dimensioniert, dass erst bei einem höheren Stand der Reinigungsflüssigkeit in der Ultraschallwanne 21 ein Gleichgewicht zwischen zulaufender Menge an Reinigungsflüssigkeit und der durch die Bohrung 23 ablaufenden Menge an Reinigungsflüssigkeit besteht. Auf diese Weise wird erreicht, dass im Betrieb des Reinigungsautomaten 1 zumindest in bestimmten Spülphasen des Reinigungsautomaten 1 das in die Ultraschallwanne 21 eingelegte Spülgut zumindest teilweise und bevorzugt vollständig von Reinigungsflüssigkeit umgeben ist. In einem derartigen Betriebszustand des Reinigungsautomaten 1 kann eine Ultraschallreinigung des Spülguts in der Ultraschallwanne 21 erfolgen.

In einer alternativen Ausgestaltung des Ultraschallmoduls 20 kann der Ablauf 23 mit einem elektrisch ansteuerbaren Ventil versehen sein. In dem Fall kann das Auffüllen der Ultraschallwanne 21 mit der Reinigungsflüssigkeit bzw. das Ablassen der Reinigungsflüssigkeit gezielt gesteuert werden. Es ist auch möglich, ein thermisch gesteuertes Ventil zu verwenden, das aufgrund der unterschiedlichen Temperaturen während des Reinigungsvorgangs den Ablauf 23 geeignet öffnet bzw. verschließt.

Zur Durchführung der Ultraschallreinigung weist das Ultraschallmodul 20 Ultraschallwandler 26 auf, die in einer geschlossenen Umhausung 25 benachbart zur Ultraschallwanne 21 angeordnet sind. Die Ultraschallwandler 26 wirken unmittelbar auf eine Trennwand 24 ein, die eine Seitenwand der Ultraschallwanne 21 ist und die Ultraschallwanne 21 von der Umhausung 25 der Ultraschallwandler 26 trennt. Im dargestellten Beispiel sind vier nebeneinander angeordnete Ultraschallwandler 26 vorgesehen. Diese Ultraschallwandler 26 sind beispielsweise piezoelektrisch arbeitende Wandler, die Schwingungen im Ultraschallbereich, beispielsweise zwischen 20 Kilohertz (kHz) und 40 kHz auf die Trennwand 24 und damit über die Reinigungsflüssigkeit auf das in der Ultraschallwanne 21 angeordnete Spülgut übertragen.

Die Ultraschallwandler 26 sind über ein elektrisches Kabel 27 mit einem elektrischen Kupplungselement 28 verbunden. Das elektrische Kupplungselement 28 und das Kabel 27 dienen der Zufuhr von elektrischer Energie für die Ultraschallwandler 26.

Die weiteren zum Betrieb der Ultraschallwandler 26 benötigten Komponenten sind in der Figur 1 dargestellt. An einer der Seitenwände 3 des Reinigungsautomaten 1 ist eine Ultraschallgeneratoreinheit 30 angeordnet, die einen Ultraschallsignalgenerator 31 aufweist, der über ein Kabel 32 mit einem Kupplungsgegenstück 33 verbunden ist. Im Betrieb des Ultraschallmoduls 20 wird vom Ultraschallsignalgenerator 31 ein elektrisches Wechselspannungssignal im genannten Frequenzbereich von 20 kHz bis etwa 40 kHz erzeugt, das über das Kabel 32 zu dem Kupplungsgegenstück 33 übertragen wird. Das Kupplungselement 28 des Ultraschallmoduls und das Kupplungsgegenstück 33 wechselwirken miteinander und übertragen die vom Ultraschallsignalgenerator 31 bereitgestellte Energie auf das Ultraschallmodul 20. Die Energieübertragung vom Kupplungsgegenstück 33 zum elektrischen Kupplungselement 28 kann durch geeignete gegen das Eindringen von Wasser geschützte Steckkontakte erfolgen. Alternativ ist eine kontaktlose, beispielsweise induktive Energieübertragung möglich. Das Kupplungsgegenstück 33 ist ebenfalls wasserdicht in der Seitenwand 3 angeordnet.

Es kann vorgesehen sein, dass nach Einschieben des Spülgutwagens 10 mit dem eingesetzten Ultraschallmodul 20 das elektrische Kupplungselement 28 händisch auf das Kupplungsgegenstück 33 aufgesteckt wird und mit diesem verbunden und ggf. verriegelt wird. Diese Verbindung bzw. Verriegelung wird dann ebenfalls händisch gelöst, bevor der Spülgutwagen zur anderen Seite des Reinigungsautomaten 1 nach erfolgter Reinigung entnommen wird. Es ist alternativ denkbar, dass das elektrische Kupplungselement 28 in eine Halterung am Spülgutwagen 10 eingesetzt wird, wobei diese Halterung so angeordnet ist, dass beim Einschieben des Spülgutwagens 10 das elektrische Kupplungselement 28 geeignet vor dem Kupplungsgegenstück 33 positioniert ist, so dass die kontaktlose Energieübertragung erfolgen kann.

Die Anordnung der Ultraschallgeneratoreinheit 30 außerhalb des Spülraums 2 und das Vorsehen von nur den Ultraschallwandlern 26 innerhalb des Ultraschallmoduls 20 bietet mehrere Vorteile. Zum einen herrschen im Spülraum 2 hohe Temperaturen, die in der Desinfektionsphase bis auf ca. 95°C und in der Trocknungsphase bis auf ca. 120°C steigen können. Derartig hohe Temperaturen sind für elektronische Komponenten des Ultraschallsignalgenerators 31 unter Umständen problematisch und können zu einer Verkürzung seiner Lebensdauer führen. Zudem erweist es sich im praktischen Betrieb des Reinigungsautomaten 1 als vorteilhaft, wenn mehrere Ultraschallmodule 20 vorhanden sind, wodurch beispielsweise ein Ultraschallmodul 20 beladen werden kann, während ein weiteres Ultraschallmodul 20 sich im Reinigungsautomaten 1 befindet. Die Anordnung der Ultraschallgeneratoreinheit 30 am Reinigungsautomaten 1 führt in diesem Fall zu einer kostengünstigen Ausgestaltung der Ultraschallmodule 20, da diese nur die Ultraschallwandler 26 und nicht die gesamten elektronischen Komponenten aufweisen müssen.

Neben der speziellen Ultraschallbehandlung erfahren in das Ultraschallmodul 20 eingelegte Instrumente auch die normalen Reinigungs-, Desinfektions- und Trocknungsschritte des Reinigungsautomaten 1. Es findet insofern eine vollständige Integration der Ultraschallreinigung in den sonstigen Reinigungsprozess statt.

### Bezugszeichenliste

- 1: Reinigungsautomat
- 2: Spülraum
- 3: Seitenwand
- 4: Boden
- 5: Decke
- 6: Tür
- 7: Spültopf
- 8: Sprüharm
- 10: Spülgutwagen
- 11: Ebene
- 12: Sprüharm
- 20: Ultraschallmodul
- 21: Ultraschallwanne
- 22: Ablagefläche
- 23: Ablauf
- 24: Trennwand
- 25: Umhausung
- 26: Ultraschallwandler
- 27: Kabel
- 28: Kupplungselement
- 30: Ultraschallgeneratoreinheit
- 31: Ultraschallsignalgenerator
- 32: Kabel
- 33: Kupplungsgegenstück

## Patentansprüche

1. Reinigungsautomat (1) für medizinische Instrumente, bei dem in einem Spülraum (2) mindestens ein Sprüharm (12) zum Versprühen einer Reinigungsflüssigkeit angeordnet ist, mit einem Ultraschallmodul (20), wobei der Reinigungsautomat (1) zur Aufnahme des aus dem Spülraum (2) entnehmbaren Ultraschallmoduls (20) eingerichtet ist,
**dadurch gekennzeichnet, dass**
das Ultraschallmodul (20) eine Ultraschallwanne (21) aufweist, die über eine Trennwand (24) mit mindestens einem Ultraschallwandler (26) gekoppelt ist, wobei der mindestens eine Ultraschallwandler (26) in einer wasserdichten Umhausung (25) angeordnet ist,
und wobei das Ultraschallmodul (20) ein Kupplungselement (28) zur Energieübertragung von einem korrespondierenden Kupplungsgegenstück (33) des Reinigungsautomaten (1) aufweist.

2. Reinigungsautomat (1) nach Anspruch 1, wobei der Reinigungsautomat (1) eine Ultraschallgeneratoreinheit (30) mit einem Ultraschallgenerator (31) zum Bereitstellen eines elektrischen Wechselspannungssignals aufweist, wobei der Ultraschallgenerator (31) außerhalb des Spülraums (2) angeordnet ist.

3. Reinigungsautomat (1) nach Anspruch 1 oder 2, bei dem das Kupplungsgegenstück (33) in den Spülraum (2) hineinragt und/oder hineinwirkt.

4. Reinigungsautomat (1) nach einem der vorhergehenden Ansprüche, bei dem die Ultraschallwanne (21) nach oben offenen ist.

5. Reinigungsautomat (1) nach einem der Ansprüche 1 bis 3, bei dem die Ultraschallwanne (21) nach oben geschlossen ist, wobei an der Ultraschallwanne (21) ein Wasserzulauf angeordnet ist.

6. Reinigungsautomat (1) nach einem der vorhergehenden Ansprüche, bei dem ein Ablauf (23) in einem unteren Bereich der Ultraschallwanne (21) angeordnet ist.

7. Reinigungsautomat (1) nach Anspruch 6, bei dem der Ablauf (23) durch eine permanent geöffnete Bohrung gebildet ist.

8. Reinigungsautomat (1) nach Anspruch 6, bei dem ein elektromagnetisch oder thermisch betätigtes Ventil vorgesehen ist, um den Ablauf (23) gesteuert zu öffnen oder zu verschließen.

9. Reinigungsautomat (1) nach einem der vorhergehenden Ansprüche, bei dem das Ultraschallmodul (20) zur Positionierung in einem Spülgutwagen (10) eingerichtet ist, der für den Reinigungsvorgang in den Reinigungsautomaten (1) eingeschoben wird.

## Claims

1. Cleaning machine (1) for medical instruments in which at least one spray arm (12) is arranged in a washing chamber (2) in order to spray a cleaning fluid, comprising an ultrasonic module (20), wherein the cleaning machine (1) is designed to receive the ultrasonic module (20), which can be removed from the washing chamber (2), **characterised in that** the ultrasonic module (20) comprises an ultrasonic bath (21) which is coupled to at least one ultrasonic transformer (26) by means of a partition wall (24), wherein the at least one ultrasonic transformer (26) is arranged in a watertight enclosure (25), and that the ultrasonic module (20) comprises a coupling element (28) for transmitting energy from a corresponding coupling counter part (33) of the cleaning machine (1).

2. Cleaning machine (1) according to claim 1, wherein the cleaning machine (1) comprises an ultrasonic generator unit (30) that has an ultrasonic generator (31) for providing an alternating-voltage electrical signal, wherein the ultrasonic generator (31) is arranged outside the washing chamber (2).

3. Cleaning machine (1) according to either claim 1 or claim 2, wherein the coupling counter part (33) protrudes and/or extends into the washing chamber (2).

4. Cleaning machine (1) according to any of the preceding claims, wherein the ultrasonic bath (21) is open at the top.

5. Cleaning machine (1) according to any of claims 1 to 3, wherein the ultrasonic bath (21) is closed at the top, wherein a water inlet is arranged in the ultrasonic bath (21).

6. Cleaning machine (1) according to any of the preceding claims, wherein an outlet (23) is arranged in a lower region of the ultrasonic bath (21).

7. Cleaning machine (1) according to claim 6, wherein the outlet (23) is formed by a hole that is permanently open.

8. Cleaning machine (1) according to claim 6, wherein an electromagnetically or thermally actuated valve is provided in order to open or close the outlet (23) in a controlled manner.

9. Cleaning machine (1) according to any of the preceding claims, wherein the ultrasonic module (20) is designed to be positioned in a washware cart (10) which is inserted into the cleaning machine (1) for the cleaning process.

## Revendications

1. Système automatique de nettoyage (1) pour instruments médicaux, selon lequel au moins un bras de pulvérisation (12) est disposé dans une chambre de lavage (2) en vue de la pulvérisation d'un liquide de nettoyage, avec un module à ultrasons (20),
dans lequel le système automatique de nettoyage (1) est aménagé en vue de la réception du module à ultrasons (20) pouvant être retiré en dehors de la chambre de lavage (2), caractérisé en ce
le module à ultrasons (20) présente un bassin à ultrasons (21) qui est couplé à au moins un transducteur à ultrasons (26) par l'intermédiaire d'une cloison de séparation (24) ;
l'au moins un transducteur à ultrasons (26) est disposé dans un habitacle (25) étanche à l'eau ; et
le module à ultrasons (20) présente un élément de couplage (28) en vue de la transmission de l'énergie par une pièce d'accouplement intermédiaire (33) correspondante du système automatique de nettoyage (1).

2. Système automatique de nettoyage (1) selon la revendication 1,
dans lequel le système automatique de nettoyage (1) présente une unité formant générateur à ultrasons (30) avec un générateur à ultrasons (31) en vue de la mise à disposition d'un signal électrique à tension alternative, dans lequel le générateur à ultrasons (31) est disposé à l'extérieur de la chambre de lavage (2).

3. Système automatique de nettoyage (1) selon la revendication 1 ou 2,
selon lequel la pièce d'accouplement intermédiaire (33) fait saillie et/ou s'étend dans la chambre de lavage (2).

4. Système automatique de nettoyage (1) selon l'une des revendications précédentes, selon lequel le bassin à ultrasons (21) est ouvert vers le haut.

5. Système automatique de nettoyage (1) selon l'une des revendications 1 à 3,
selon lequel le bassin à ultrasons (21) est fermé vers le haut, dans lequel une arrivée d'eau est disposée au niveau du bassin à ultrasons (21).

6. Système automatique de nettoyage (1) selon l'une des revendications précédentes, selon lequel une sortie d'eau (23) est disposée dans une zone inférieure du bassin à ultrasons (21).

7. Système automatique de nettoyage (1) selon la revendication 6,
selon lequel la sortie d'eau (23) est formée par un trou ouvert de manière permanente.

8. Système automatique de nettoyage (1) selon la revendication 6,
selon lequel une vanne dotée d'une commande électromagnétique ou thermique est prévue en vue de piloter l'ouverture ou la fermeture de la sortie d'eau (23).

9. Système automatique de nettoyage (1) selon l'une des revendications précédentes,
selon lequel le module à ultrasons (20) est aménagé en vue de sa mise en place dans un chariot d'articles à nettoyer (10), lequel est inséré dans le système automatique de nettoyage (1) pour le processus de nettoyage.
